# EUROPÄISCHE PATENTANMELDUNG

(11) **EP 1 927 325 A1**
(43) Veröffentlichungstag der Anmeldung: **04.06.2008**
(21) Anmeldenummer: 06024906.7
(22) Anmeldetag: 01.12.2006
(51) Int. Cl.: A61C 8/00

(54) **Implantat und Verfahren zur Oberflächenmodifikation eines Implantats**

(71) Anmelder: ZL Microdent-Attachment GmbH & Co. KG, 58339 Breckerfeld (DE)
(72) Erfinder: Böschemeyer, Thomas, 58313 Herdecke (DE); Clostermann, Volkhard-Hagen, 58097 Hagen (DE); Graf, Hans-Ludwig, Prof. Dr. med. dent., 04451 Panitzsch (DE)
(74) Vertreter: Kötter, Ulrich

(57) **Zusammenfassung**

Die Erfindung betrifft ein Implantat (1) sowie einen Implantataufbau (3), der zumindest bereichsweise mit einer annähernd zahnfarbenen, mundbeständigen Materialschicht (2) versehen ist. Die Erfindung betrifft weiterhin ein Verfahren zur Oberflächenmodifikation eines Implantats (1), wobei das Implantat (1) zumindest bereichsweise mit einer annähernd zahnfarbenen, mundbeständigen Materialschicht (2) versehen wird.

## Beschreibung

Die Erfindung betrifft ein Implantat nach dem Oberbegriff des Patentanspruchs 5 sowie einen zahnmedizinischen lmplantataufbau nach dem Oberbegriff des Patentanspruchs 7. Die Erfindung betrifft weiterhin ein Verfahren zur Oberflächenmodifikation eines Implantats nach dem Patentanspruch 1 sowie die Verwendung der Plasma-Immersions-Ionenimplantation (PIII) zur Oberflächenmodifikation eines Implantats.

Der Einsatz zahnmedizinischer Implantate ist fester Bestandteil der Zahnmedizin. Derartige Implantate sind in den unterschiedlichsten Ausführungen bekannt und werden entweder in künstlich in einen Kiefer eingebrachte Öffnungen eingeschraubt oder - sofern der Knochenkontaktbereich eines Implantats der Wurzelform eines extrahierten Zahnes nachgebildet wurde - in das so entstandene, leere Zahnfach eingesetzt. Nach vollständiger Einheilung des Implantats wird in dieses ein Aufbau eingeschraubt, an dem eine Zahnkrone befestigt ist. Auf Grund seiner biokompatiblen Eigenschaften ist das Implantat sowie der Aufbau dabei aus Titan hergestellt. Die an dem Aufbau befestigte Zahnkrone ist in der Regel aus Zirkoniumoxid/-dioxid hergestellt. Die Farbe des Zirkoniumoxids/-dioxids und die biotechnischen Charakteristiken dieses Materials ermöglichen qualitativ hochwertige und ästhetische Zahnrekonstruktionen.

Nachteilig an den bekannten Implantatsystemen ist, dass im Falle eines Zahnfleisch- und / oder Knochenrückgangs das titanfarbene Implantat zum Vorschein kommt, welches metallfarben schimmert und vielfach als ästhetisch störend empfunden wird. Es ist bekannt, den Halsbereich eines Implantats zu verjüngen und mit einer Hülse aus Zirkoniumoxid/-dioxid zu versehen, welche mittels bekannter Fügeverfahren an dem Implantat befestigt wird. Diese Lösung erweist sich als sehr aufwendig. Darüber hinaus besteht die Gefahr, dass sich die Keramikhülse im Laufe der Zeit lockert. Eine weitere Lösung besteht darin, sowohl das Implantat als auch dem Implantataufbau aus Zirkoniumoxid/-dioxid herzustellen. Diese Lösung hat sich jedoch in der Praxis als nachteilig erwiesen, dass die Nachbearbeitung des keramischen Materials durch den Zahnarzt nahezu ausgeschlossen ist.

Hier will die Erfindung Abhilfe schaffen. Der Erfindung liegt die Aufgabe zu Grunde, ein Verfahren zur Oberflächenmodifikation eines Implantats zu schaffen, welches das Hervortreten metallfarbener Teile bei der Verwendung von Titanimplantaten vermeidet. Gemäß der Erfindung wird diese Aufgabe durch die Merkmale des Patentanspruchs 1 gelöst. Die Aufgabe wird darüber hinaus auch durch die Merkmale des Patentanspruchs 10 gelöst.

Mit der Erfindung ist ein Verfahren zur Oberflächenmodifikation eines Implantats geschaffen, welches das Hervortreten metallfarbener Teile bei der Verwendung von Titanimplantaten vermeidet.

In Weiterbildung der Erfindung wird die Materialschicht mittels physikalischer Gasphasenabscheidung aufgebracht. Bei diesem sogenannten PVD-Verfahren (physical vapour deposition) wird die Materialschicht durch physikalische Abscheidung aus der Dampfphase aufgebracht. Dieses insbesondere aus dem Bereich der Werkzeugbeschichtung bekannte Verfahren ermöglicht eine hohe Haftfestigkeit der Materialschicht mit dem Implantat sowie einen gleichmäßigen Schichtaufbau.

In weiterer Ausgestaltung der Erfindung wird die Materialschicht mittels Plasma-immersions-Ionenimplantation (PIII) aufgebracht. Hierbei wird das Werkstück in das Plasma eingetaucht, sodass die zu behandelnden Flächen vollständig vom Plasma umgeben sind. Durch das Anlegen einer negativen Hochspannung werden die Ionen aus dem Plasma zum Werkstück beschleunigt und implantiert. Da hierbei die gesamte Fläche gleichzeitig implantiert wird, ist das Verfahren gerade dort, wo eine hohe Dosis der Implantation gefordert wird, besonders produktiv. Die Stromdichte der implanlierten Ionen ist von den Plasmaparametern und der gewählten Implantationsspannung abhängig und liegt während der Implantation in der Größenordnung von 1-10 mA/cm2. Um die Aufheizung des Werkstücks zu begrenzen wird zur Implantation eine gepulste Hochspannung, die in so genannten Modulatoren erzeugt wird, eingesetzt. Die üblichen Pulsdauern liegen zwischen 2 und 100 µs, bei einer Wiederholfrequenz von wenigen Hz bis kHz. Der Vorteil dieses Beschichtungsverfahrens liegt bei dem nichtthermischen Energieeintrag der beschleunigten Ionen und der gleichzeitigen atomaren Durchmischung der Implantationszone. Dadurch ergeben sich sehr gute haftende Verbindungen zwischen Substrat und aufwachsender Schicht.

In alternativer Ausgestaltung der Erfindung wird die Materialschicht mittels chemischer Gasphasenabscheidung aufgebracht. Das sogenannte CVD-Verfahren (chemical vapour deposition) funktioniert ähnlich wie das PVD-Verfahren, das heißt die Feststoffe werden zunächst aus der Dampfphase abgeschieden und anschließend mittels chemischer Reaktion in verschleißfeste Verbindungen umgewandelt. Für temperaturempfindliche Bauteile hat sich insbesondere das Plasma-CVD-Verfahren bewehrt, da bei Temperaturen >300°C durch elektrische Entladung im thermischen Plasma oder mit kaltem Plasma bei vermindertem Druck gearbeitet wird. Auch das CVD-Verfahren ermöglicht eine hohe Haftfestigkeit der Materialschicht mit dem Implantat sowie einen gleichmäßigen Schichtaufbau.

Bevorzugt ist die Materialschicht eine Zirkonoxid/-dioxid- oder eine Aluminiumoxid/-dioxidschicht. Diese Materialien sind besonders biokompatibel und lassen sich hinsichtlich ihrer Farbgebung besonders gut der natürlichen Zahnfarbe anpassen.

Der Erfindung liegt weiterhin die Aufgabe zu Grunde, ein zahnmedizinisches Implantat zu schaffen, welches auch im Falle eines Zahnfleisch- oder Knochenrückgangs ein Hervortreten metallfarbener Teile vermeidet. Gemäß der Erfindung wird diese Aufgabe durch die Merkmale des kennzeichnenden Teils des Patentanspruchs 5 gelöst.

Mit der Erfindung ist ein zahnmedizinisches Implantat geschaffen, das auch im Falle eines Zahnfleisch- und Knochenrückgangs ein Hervortreten metallfarbener Teile vermeidet.

Bevorzugt ist die Materialschicht eine Zirkonoxid/-dioxidschicht oder eine Aluminiumoxid/-dioxidschicht. Diese Materialien sind besonders biokompatibel und lassen sich hinsichtlich ihrer Farbgebung besonders gut der natürlichen Zahnfarbe anpassen.

Der Erfindung liegt weiterhin die Aufgabe zu Grunde einen zahnmedizinisches Implantataufbau zur Befestigung an einem zahnmedizinischen Implantat zu schaffen, der auch im Falle eines Zahnfleisch- und / oder Knochenrückgangs ein Hervortreten metallfarbener Teile vermeidet. Gemäß der Erfindung wird diese Aufgabe durch die Merkmale des kennzeichnenden Teils des Patentanspruchs 7 gelöst.

Mit der Erfindung ist ein zahnmedizinischer Implantataufbau zur Befestigung an einem zahnmedizinischen Implantat geschaffen, der auch im Falle eines Zahnfleisch- und / oder Knochenrückgangs ein Hervortreten metallfarbener Teile vermeidet.

Vorteilhaft ist die Materialschicht eine Zirkonoxid/-dioxidschicht oder eine Aluminiumoxid/-dioxidschicht. Diese Materialien sind besonders biokompatibel und lassen sich hinsichtlich ihrer Farbgebung besonders gut der natürlichen Zahnfarbe anpassen.

Andere Weiterbildungen und Ausgestaltungen sind in den übrigen Unteransprüchen angegeben. Ein Ausführungsbeispiel der Erfindung ist in den Zeichnungen dargestellt und wird nachfolgend im Einzelnen beschrieben. Es zeigen:
- Figur 1: ein zahnmedizinisches Implantat und
- Figur 2: das zahnmedizinische Implantat aus Figur 1 mit zahnmedizinischem Implantataufbau.

Das als Ausführungsbeispiel gewählte Implantat 1 ist aus Titan hergestellt und besteht im Wesentlichen aus einem Grundkörper 11 mit angeformtem Anschlussstück 12. Der Grundkörper 11 ist außen umlaufend mit einem Außengewinde versehen, auch das angrenzende Anschlussstück 2 weist außen umlaufend ein Außengewinde auf. Innerhalb des Anschlussstücks 12 ist ein Innenvielkant 13 angeordnet. Das Anschlussstück 12 ist umlaufend mit einer Zirkondioxidschicht 2 versehen. Im Ausführungsbeispiel wurde die Zirkondioxidschicht mittels Plasma-Immersions-Ionenimplantation (PIII) auf den Titankörper aufgebracht.

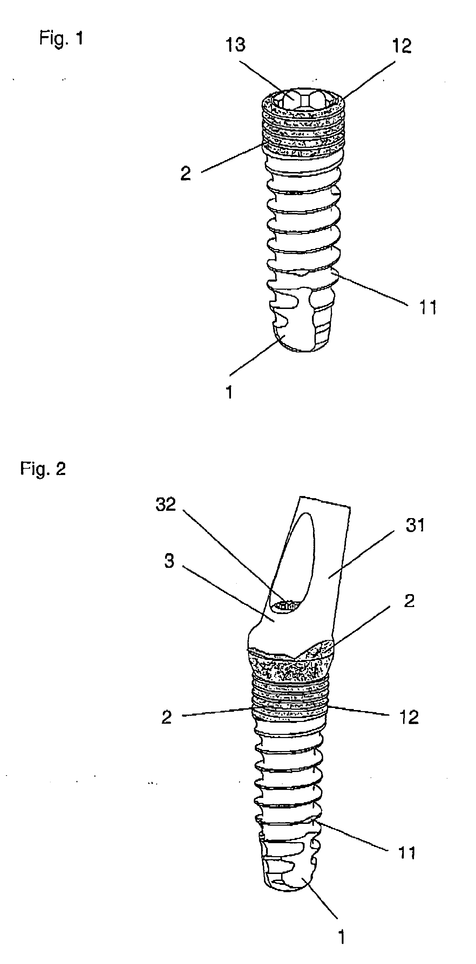

Im Ausführungsbeispiel gemäß Figur 2 ist in das Implantat 1 ein Aufbau 3 eingeschraubt. Der Aufbau 3 ist ebenfalls aus Titan hergestellt und dient der Befestigung einer - nicht dargestellten - Zahnkrone. Hierzu weist der Aufbau 3 einen Anschlusspfosten 31 auf. Der Aufbau 3 ist über eine Vielkantschraube 32 mit dem Implantat 1 verschraubt. Der Aufbau 3 ist an seinem an das Anschlussstück 12 des Implantats 1 angrenzenden Bereich umlaufend mit einer Zirkondioxidschicht 2 versehen. Auch diese Zirkondioxidschicht wurde auf den Titankörper mittels Plasma-Immersions-Ionenimplaniation (Plll) aufgebracht.

## Patentansprüche

1. Verfahren zur Oberflächenmodifikation eines Implantats, wobei das Implantat (1) zumindest bereichsweise mit einer annähernd zahnfarbenen, mundbeständigen Materialschicht (2) versehen wird.

2. Verfahren nach Anspruch 1, **dadurch gekennzeichnet, dass** die Materialschicht (2) mittels physikalischer Gasphasenabscheidung (PVD-Verfahren) aufgebracht wird.

3. Verfahren nach Anspruch 1, **dadurch gekennzeichnet, dass** die Materialschicht (2) mittels Plasma-Immersions-Ionenimplantation (PIII) aufgebracht wird.

4. Verfahren nach Anspruch 1, **dadurch gekennzeichnet, dass** die Materialschicht (2) mittels chemischer Gasphasenabscheidung (CVD-Verfahren) aufgebracht wird.

5. Verfahren nach einem der vorgenannten Ansprüche, **dadurch gekennzeichnet, dass** die Materialschicht (2) eine Zirkonoxid-, eine Zirkondioxid-, eine Aluminiumoxid- oder eine Aluminiumdioxidschicht ist.

6. Zahnmedizinisches Implantat, umfassend einen zapfenförmigen Grundkörper (1) mit einem Außengewinde zur Befestigung in einem Kieferknochen, **dadurch gekennzeichnet, dass** das Implantat (1) zumindest bereichsweise mit einer annähernd zahnfarbenen, mundbeständigen Materialschicht (2) versehen ist.

7. Implantat nach Anspruch 6, **dadurch gekennzeichnet, dass** die Materialschicht (2) eine Zirkonoxid-, eine Zirkondioxid-, eine Aluminiumoxid- oder eine Aluminiumdioxidschicht ist.

8. Zahnrnedizinischer Implantataufbau zur Befestigung an einem zahnmedizinischen Implantat, umfassend wenigstens einen Anschlusspfosten (31) und eine Zahnkrone, **dadurch gekennzeichnet, dass** der Implantataufbau zumindest bereichsweise mit einer annähernd zahnfarbenen, mundbeständigen Materialschicht (2) versehen ist.

9. Implantataufbau nach Anspruch 8, **dadurch gekennzeichnet, dass** die Materialschicht (2) eine Zirkonoxid-, eine Zirkondioxid-, eine Aluminiumoxid- oder eine Aluminiumdioxidschicht ist.

10. Verwendung der Plasma-Immersions-Ionenimplantation (PIII) zur Oberflächenmodifikation eines zahnmedizinischen Implantats (1), wobei das Implantat (1) mit einer annähernd zahnfarbenen, mundbeständigen Materialschicht (2) versehen wird.
